# EUROPEAN PATENT APPLICATION

(11) **EP 4 498 383 A1**
(43) Date of publication of application: **29.01.2025**
(21) Application number: 23187481.9
(22) Date of filing: 25.07.2023
(51) Int. Cl.: G16H 40/60, G16H 30/20, G16H 30/40, G16H 50/70, G16H 40/40, A61B 8/00

(54) **AUTOMATED MODIFICATION OF IMAGING SYSTEM SETTINGS FOR A MEDICAL IMAGING SYSTEM**

(71) Applicant: Koninklijke Philips N.V., 5656 AG Eindhoven (NL)
(72) Inventor: KAUSHAL, Nitesh, Eindhoven (NL); KOTHAWALA, Aliarshad Aameer, Eindhoven (NL); SETH, Subhendu, 5656AG Eindhoven (NL); ASWATHA, Shashaank Mattur, Eindhoven (NL); GOVINAHALLISATHYANARAVANA, Sushanth, Eindhoven (NL); M, Gurunath Reddy, Eindhoven (NL); VAJINEPALLI, Pallavi, Eindhoven (NL)
(74) Representative: Philips Intellectual Property & Standards

(57) **Abstract**

A mechanism for determining one or more imaging system settings for a medical imaging system. Initial imaging system settings are defined using previous imaging system settings for performing a medical imaging procedure on the subject to be imaged. The initial imaging system settings are modified using a machine-learning method or algorithm to output imaging system settings to be used.

## Description

### FIELD OF THE INVENTION

The present invention relates to the field of medical imaging systems, and in particular, to the selection of imaging system settings of a medical imaging system.

### BACKGROUND OF THE INVENTION

In the field of medicine, particularly diagnostic medicine, there is an increasing use of medical imaging systems to capture medical imaging data. Medical imaging data is particularly useful for assessing the condition of an imaged subject, e.g., for the purposes of diagnosis, assessment and/or monitoring.

Example medical imaging systems include ultrasound imaging systems, magnetic resonance (MR) imaging systems, X-ray imaging systems, computerized tomography (CT) imaging systems, position emission tomography (PET) imaging systems and so on.

In performing a medical imaging procedure using a medical imaging system, correct selection of medical system settings, also known as medical system parameters, is important to achieve a dialogistically or clinically acceptable image. Examples of medical system settings, for an ultrasound imaging system, include maximum depth of scan, focus, dynamic range, scan mode, time gain compensation curve and digital gain. Other examples of medical system settings, and those suitable for other medical imaging systems, are known to the skilled person.

It is common for an operator of the medical imaging system to manually set the medical system parameters. However, this necessitates training for such operators and can often prove operator-specific, e.g., as each operator may have their own preferences for characteristics of medical imaging data. In some approaches, a medical imaging system may make use of pre-programmed defaults for the medical system parameters, which may be optionally adjustable by the operator.

There is an ongoing desire to improve the consistency, reliability and comparability of medical imaging data.

### SUMMARY OF THE INVENTION

The invention is defined by the claims.

According to examples in accordance with an aspect of the invention, there is provided a computer-implemented method for defining one or more imaging system settings for a medical imaging system to use in performing a medical imaging procedure on a subject.

The computer-implemented method comprises: obtaining anatomical information that indicates one or more anatomical features of the subject to undergo the medical imaging procedure; obtaining, from a database and using the anatomical information, one or more imaging system settings used to perform a previous medical imaging procedure on the indicated one or more anatomical features of the subject; and tuning the obtained one or more image system settings using a machine-learning method.

The present disclosure proposes a technique for improved determination of imaging system settings using a machine-learning method. In particular, old or previous settings for the medical imaging system are retrieved or otherwise obtained from a database, and used as the baseline or initial conditions, for modifying the settings using the machine-learning method. The previous settings are settings used for the same subject (e.g., patient), such that the modification makes use of subject-specific settings, and for the same anatomical feature(s) of said subject.

The proposed approach has been shown to improve the performance of machine-learning led modification or selection of imaging system settings. In particular, the proposed approach, for iterative modification techniques, has been identified as reducing the number of iterations required to perform machine-learning method led modification or selection of imaging system settings, e.g., compared to approaches that make use of default or randomized imaging system settings as the baseline for machine-learning method modification.

The proposed approach provides a technique for more efficiently identifying imaging system settings for performing a medical imaging procedure that exploits historic image system settings, e.g., stored in a database. Thus, a new route of communication is used (e.g., to a previously unused part of a system) in order to improve the performance of a technique for identifying imaging system settings. This approach thereby provides values for parameters used to control a specific technical system, namely a medical imaging system.

The proposed approach also restricts the (initial) imaging system settings to those that were used to image a same region of interest (i.e., the same one or more anatomical features) of a previous medical imaging procedure. This approach has been shown to further improve the performance of an imaging system setting selection and/or reduce the number of iterations required to perform a machine-learning method led iterative modification or selection of imaging system settings.

The anatomical information may identify: the one or more anatomical features; and/or a view of the subject.

The step of tuning the obtained one or more image system settings may comprise performing an imaging system setting modification process comprising: obtaining, from the medical imaging system, medical imaging data obtained using the one or more imaging system settings; processing the medical imaging data, using the machine-learning method, to identify one or more changes to be made to the one or more imaging system settings; and modifying the one or more imaging system settings using the identified one or more changes.

The step of tuning the obtained one or more image system settings may comprise iteratively performing the imaging system setting modification process until one or more termination conditions are met.

The one or more termination conditions may include: a quality measure of the medical imaging data meeting one or more predetermined conditions; the imaging setting modification process being performed no less than a predetermined number of times; a measure of a difference between the imaging system settings for a current iteration of the imaging setting modification process and the imaging system settings for an immediately previous iteration of the imaging setting modification process being less than a predetermined value; and/or an override indicator being provided. Other examples will be apparent to the appropriately skilled person.

The step of obtaining anatomical information may comprise obtaining, from the medical imaging system, initial imaging data of the subject; and processing the initial imaging data to identify the anatomical information. Thus, the anatomical information may be derived from initial imaging data of the subject.

The initial imaging data may be obtained by the medical imaging system using randomized or pseudo-randomized imaging system settings. In other examples, the initial imaging data may be obtained by the medical imaging system using default imaging system settings and/or user-defined imaging system settings.

The anatomical information may identify the one or more anatomical features represented in the initial imaging data. In some examples, the anatomical information identifies the view of the subject represented by the initial imaging data.

The step of processing the initial imaging data may comprise processing the initial imaging data using a second machine-learning method. Thus, a second machine-learning method may be used to process the initial imaging data to identify the anatomical data, e.g., using one or more classifiers, segmentation algorithms and so on.

The step of obtaining, from a database, one or more imaging system settings may comprise: obtaining purpose information that indicates a purpose for medical imaging data to be produced by the medical imaging system performing the medical imaging procedure; and using the purpose information to obtain, from the database, one or more imaging system settings used by the medical imaging system to perform the previous medical imaging procedure, wherein the previous medical imaging procedure had a same purpose as the medical imaging procedure.

This approach restricts the (initial) imaging system settings to those that were used for performing a medical imaging procedure having a same purpose as the (current) medical imaging procedure. This approach has been shown to further reduce the number of iterations required to perform machine-learning method led modification or selection of imaging system settings.

More particularly, it has been identified that different imaging system settings will be used by a same medical imaging system to perform medical imaging procedures having different purposes, e.g., to produce different types of medical imaging data.

The purpose information may be defined by a user input at a user interface. Other approaches include defining the purpose information based on medical history data, notes by a clinician in an electronic medical record for the subject and/or other data contained in the electronic medical record.

The one or more imaging system settings may comprise one or more settings for capturing medical imaging data. Other suitable examples of imaging system settings include settings for processing (raw) medical imaging data and/or enhancing (raw) medical imaging data.

The medical imaging system may be an ultrasound imaging system. Accordingly, the medical imaging data may comprise medical ultrasound data. Proposed approaches are particularly advantageous for use with ultrasound imaging systems, as the tuning of settings for an ultrasound imaging system significantly affects or influences the quality and value of the medical imaging data thereby produced. The effect of different medical imaging settings is less pronounced in other forms of medical imaging system. However, it will be appreciated that the proposed approach can be applied to any other form of medical imaging system, examples of which are well known in the art.

The step of tuning the obtained one or more image system settings may further comprise using the purpose information to define the number of image system settings that are modified and/or the amount to which the image system settings are modified using the machine-learning method.

There is also proposed a computer program product comprising computer program code means which, when executed on a computing device having a processing system, cause the processing system to perform all of the steps of any herein disclosed method.

There is also proposed a processing system for defining one or more imaging system settings for a medical imaging system to use in performing a medical imaging procedure on a subject.

The processing system is configured to: obtain anatomical information that indicates one or more anatomical features of the subject to undergo the medical imaging procedure; obtain, from a database and using the anatomical information, one or more imaging system settings used to perform a previous medical imaging procedure on the indicated one or more anatomical features of the subject; and tune the obtained one or more image system settings using a machine-learning method.

The processing system may be modified to carry out the functions of any herein disclosed method and vice versa.

There is also proposed an imaging system comprising the processing system; and the medical imaging system configured to use the one or more imaging system settings defined by the processing system to perform the medical imaging procedure and generate medical imaging data.

The imaging system may be further configured to store, in the database, the one or more imaging settings used by the medical imaging system to perform the medical imaging procedure and generate the medical imaging data.

The imaging system may further comprise a user interface configured to provide a visual representation or display of the medical imaging data produced/generated by the medical imaging system.

The medical imaging system may be an ultrasound imaging system comprising an ultrasound probe configured to capture, as the medical imaging data, medical ultrasound data.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a contextual workflow;
Fig. 2 is a flowchart illustrating a proposed method;
Fig. 3 illustrates an approach for tuning imaging system settings;
Fig. 4 illustrates a step for obtaining anatomical information;
Fig. 5 illustrates another step for obtaining initial imaging system settings;
Fig. 6 illustrates a processing system; and
Fig. 7 illustrates an imaging system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for determining one or more imaging system settings for a medical imaging system. Initial imaging system settings are defined using previous imaging system settings for performing a medical imaging procedure on the subject to be imaged. The initial imaging system settings are modified using a machine-learning method or algorithm to output imaging system settings to be used.

In the context of the present disclosure, reference to any function that processes an imaging system setting comprises processing one or more values of an imaging system parameter or setting.

In the context of the present disclosure, medical imaging data is data produced by a medical imaging system. The medical imaging data may, for instance, comprise one or more 2D or 3D medical images of a region of interest of a subject, as is well known in the art.

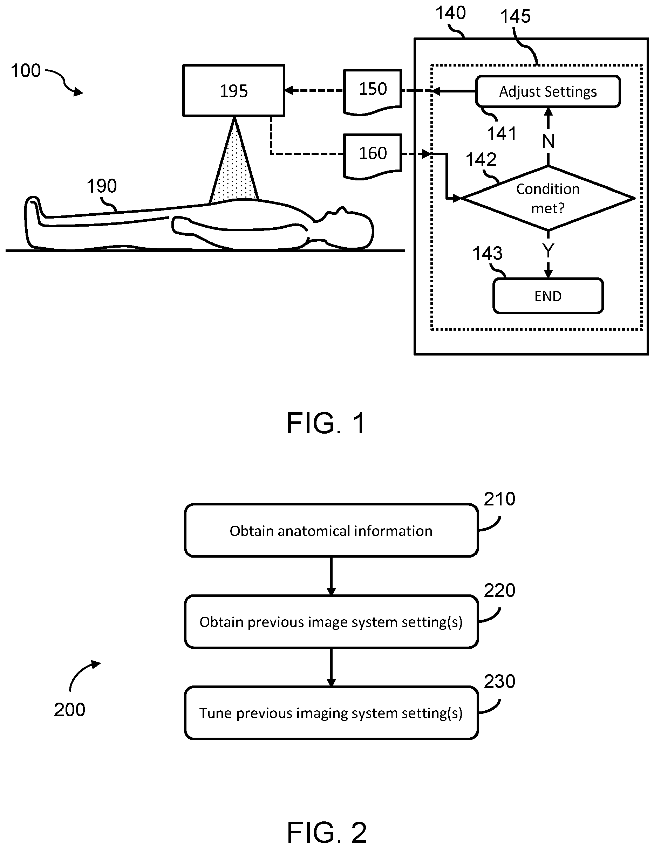

Fig. 1 conceptually illustrates a workflow 100 in which embodiments can be implemented, for the sake of improved contextual understanding.

A medical imaging procedure is performed on a subject 190 using a medical imaging system 195. Examples of medical imaging systems, and corresponding procedures, are well known in the art. Example medical imaging systems include ultrasound imaging systems, magnetic resonance (MR) imaging systems, X-ray imaging systems, computerized tomography (CT) imaging systems, position emission tomography (PET) imaging systems and so on.

In order to perform the medical imaging procedure, one or more imaging system settings 150 are defined for the medical imaging system 195. It is traditional for the (values of the) imaging system settings to be defined by an operator of the medical imaging system, e.g., during the course of the medical imaging procedure.

An alternative approach for defining the (values of the) imaging system settings is to make use of a machine-learning method, e.g., carried out by a processing system 140. One approach for using a machine-learning method is to perform an imaging system setting modification process 145 to modify the value(s) of one or more imaging system settings. The imaging system setting modification process is configured to modify initial imaging system settings.

The setting modification process may, for instance, process 141 medical imaging data 160 (obtained by the medical imaging system 195 with existing imaging system settings 150) using the machine-learning method to identify one or more changes to make to the imaging system settings. The changes are then made to the imaging system settings.

In some examples, the imaging system setting modification process 145 is performed iteratively in order to modify the one or more imaging system settings. Thus, in this approach, the iterative setting modification process 145 is repeated until one or more termination conditions are met 142. Once the condition(s) is/are met, the iterative modification process ends 143.

In other examples, the imaging system setting modification process is a single-step procedure, i.e., is not iteratively performed. Such approaches may, for instance, make use of a machine-learning algorithm configured as an echo state network; a random vector functional link; an extreme learning machine or a decision tree.

It will be appreciated that, typically, the value(s) for the one or more imaging system settings 150 are, before the setting process 145 is performed, initialized to corresponding one or more initial values. Typically, the initial value(s) is/are randomized or pseudorandomized.

The proposed approach proposes to instead set the initial values for the one or more imaging system settings based on previous imaging system settings used to perform a previous medical imaging procedure on one or more indicated anatomical features of the subject. Thus, the initial conditions for the iterative setting modification process are defined based on previous imaging system settings of that subject and, more particularly, those used to image indicated one or more anatomical features of said subject. This has been identified as significantly reducing the number of iterations to arrive at values for the imaging system settings that exhibit improved production of medical imaging data.

Fig. 2 is a flowchart illustrating a computer-implemented method 200 for defining one or more imaging system settings for a medical imaging system to use in performing a medical imaging procedure on a subject. The medical imaging system may, for instance, be an ultrasound imaging system.

The imaging system setting(s) may comprise one or more settings for capturing medical imaging data. The precise type and form of setting may, for instance, depend upon the (type of) medical imaging procedure to be performed and/or the medical imaging system that performs the medical imaging procedure. Other suitable examples of imaging system settings include settings for processing (raw) medical imaging data and/or enhancing (raw) medical imaging data.

Examples of medical imaging system settings, for an ultrasound imaging system, include maximum depth of scan, focus, dynamic range, scan mode, time gain compensation curve and digital gain. Other examples of medical imaging system settings, and those suitable for other medical imaging systems, are known to the skilled person.

The method 200 comprises a step 210 of obtaining anatomical information that indicates one or more anatomical features of the subject to undergo the medical imaging procedure. Step 210 may, for instance, comprise receiving the anatomical information from a user input and/or an electronic medical record or database (e.g., indicating a schedule for the subject to undergo imaging of a particular anatomical feature). Another example approach for performing step 210 using initial imaging data is described later in this document.

In particular examples, the anatomical information may identify the one or more anatomical features represented in the initial imaging data. In some examples, the anatomical information identifies the view of the subject represented by the initial imaging data.

The method 200 also comprises a step or process 220 of obtaining, from a database and using the anatomical information, one or more imaging system settings used to perform a previous medical imaging procedure on the indicated one or more anatomical features of the subject. The previous medical imaging procedure is a same type of medical imaging procedure (e.g., ultrasound or CT scan) as the medical imaging procedure to be performed.

Thus, step 220 comprises using the anatomical information to obtain, from the database, one or more imaging system settings used by the medical imaging system to perform the previous medical imaging procedure, wherein the previous medical imaging procedure was performed on the same one or more anatomical features as represented in the initial imaging data.

In this way, the (initial) imaging system setting(s) is/are defined based on the imaging system setting(s) used to image a same anatomical region or area as imaged in a previous medical imaging procedure. This has been identified as improving the speed of convergence of the imaging system setting(s), e.g., improved speed of optimization. This approach also increases the quality of the medical imaging data 160 produced using the (unmodified/original) imaging system settings, such that any obtained medical imaging data for tuning the previous imaging system settings is improved (e.g., more likely to converge or approach imaging system settings that produce medical imaging data having desired characteristics).

In some examples, the database used in process 220 may comprise one or more sets of imaging system settings for a single subject. For instance, the database may be an electronic medical record of the single subject. This approach avoids the need to search for or otherwise identify imaging system settings of a particular subject.

In other examples, the database used in process 220 may comprise a plurality of different sets of imaging system settings, e.g., for a plurality of different subjects. In particular, each set of imaging system settings may comprise a subject identifier that identifies the subject who underwent the medical imaging procedure. Process 220 may comprise identifying the set(s) of imaging system settings associated with a particular subject, e.g., by identifying the set(s) having the subject identifier associated with the subject to undergo the medical imaging procedure. Accordingly, process 220 may comprise obtaining a subject identifier (e.g., a subject name, number or the like) and using the subject identifier to identify the set(s) of imaging system settings.

The set(s) of imaging system settings may then be processed using the anatomical information to identify the set(s) of imaging system settings that were used to image the same anatomical feature(s) as to undergo the medical imaging procedure.

It is possible that more than one set of imaging system setting(s) will be associated with the subject and the same anatomical feature(s). In these circumstances, the most recent set of imaging system setting(s) - e.g., those used with the most recently performed medical imaging procedure - may be selected in process 210. Thus, each set of imaging system settings may be associated with a timestamp or the like.

As previously identified, the previous medical imaging procedure is a same type of medical imaging procedure (e.g., ultrasound or CT scan) as the medical imaging procedure to be performed. If the database comprises imaging system settings for different types of medical imaging procedures, then a further filter or restriction may be that the set(s) of medical imaging system settings are selected to match the type of medical imaging system/procedure to be performed on the subject.

Further approaches for narrowing down which set(s) of imaging system settings are obtained in step 210 are described later in this document, e.g., making use of purpose information.

The method 200 also comprises a step 230 of tuning the obtained one or more imaging system settings using a machine-learning method. Thus, step 230 may effectively comprise performing an imaging system setting modification process. Step 230 may, for instance, be performed using the iterative imaging system setting modification process previously described, or by using a single-step (i.e., non-iterative) imaging system setting modification process. Other approaches will be apparent to the skilled person.

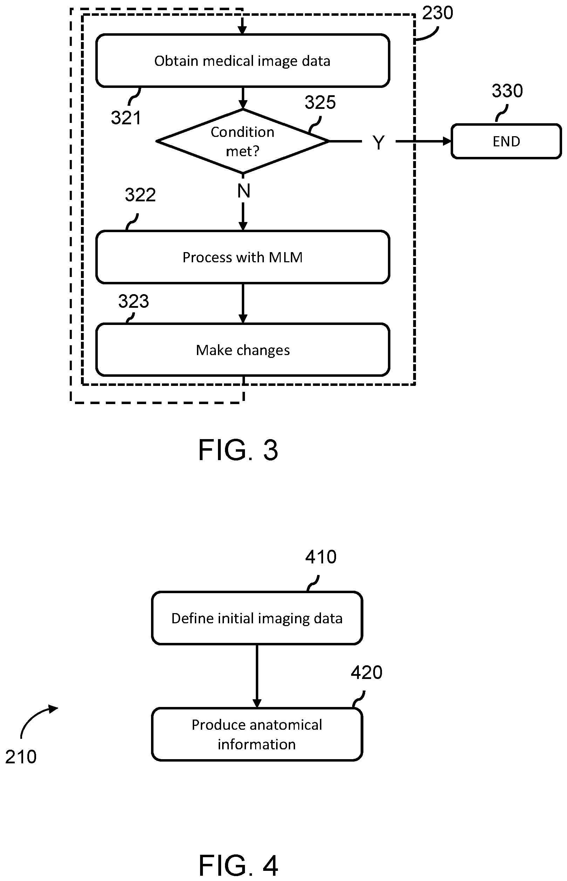

Fig. 3 provides one embodiment of an imaging system setting modification process 230.

The exemplified imaging system setting modification process 230 comprises a step 321 of obtaining, from the medical imaging system, medical imaging data obtained using the one or more imaging system settings. The exemplified imaging system setting modification process 230 also comprises a step 322 of processing the medical imaging data, using a machine-learning method, to identify one or more changes to be made to the one or more imaging system settings. The exemplified imaging system setting modification process 230 also comprises a step 323 of modifying the one or more imaging system settings using the identified one or more changes.

In some examples, the process 230 is iteratively repeated (as indicated with the dashed line) until one or more termination conditions are met. Accordingly, the imaging system setting modification process 220 may also comprise a step 325 of determining whether or not the one or more termination conditions are met. Responsive to a positive determination in step 325, the process 230 ends in step 330. Otherwise, the process 230 continues.

Examples of suitable termination conditions will be readily apparent to the skilled person.

One example termination condition is that the medical imaging data meets one or more predetermined conditions (e.g., a desired quality, a desired sharpness, a desired contrast level, a desired signal-to-noise rail etc.). In particular, the termination condition may be that a metric or measure of the medical imaging data meets one or more predetermined conditions. Approaches for determining or quantifying a quality and/or property of medical imaging data are well known in the art. Any such measures or values may be labelled a "quality measure".

Campanella, Gabriele, et al. "Towards machine learned quality control: A benchmark for sharpness quantification in digital pathology." Computerized Medical Imaging and Graphics 65 (2018): 142-151 provides one example technique for quantifying a sharpness of medical images. Zhang, Zhicheng, et al. "Can signal-to-noise ratio perform as a baseline indicator for medical image quality assessment." IEEE Access 6 (2018): 11534-11543 describes an approach for assessing a signal-to-noise ratio of a medical image. Tripathi, Abhishek Kumar, Sudipta Mukhopadhyay, and Ashis Kumar Dhara. "Performance metrics for image contrast." 2011 International Conference on Image Information Processing. IEEE, 2011 discloses approaches for quantifying a contrast in imaging data. Other approaches for determining any suitable metric will be readily apparent to the skilled person.

Another example termination condition is that the imaging system settings have changed by less than a predetermined measure for a predetermined number of iterations. Yet another example termination condition is that a predetermined number of iterations of the setting modification process have been performed. Yet another example termination condition is that an override signal or trigger is received. A wide variety of other examples will be readily apparent to the skilled person.

Thus, the one or more termination conditions may comprise one or more of the imaging setting modification process being performed no less than a predetermined number of times; a measure of a difference between the imaging system settings for a current iteration of the imaging setting modification process and the imaging system settings for an immediately previous iteration of the imaging setting modification process being less than a predetermined value; and/or an override indicator being provided.

The proposed approach makes use of a machine-learning method to identify one or more changes to make to the imaging system settings. A machine-learning method is any self-training algorithm that processes input data in order to produce or predict output data. Here, the input data comprises medical imaging data and/or imaging system settings and the output data comprises changes to the imaging system settings.

Suitable machine-learning methods for being employed in the present invention will be apparent to the skilled person. Examples of suitable machine-learning methods include decision tree algorithms and artificial neural networks. Other machine-learning methods such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning method are well known. Typically, such methods comprise obtaining a training dataset, comprising training input data entries and corresponding training output data entries. The training output data entries may, for instance, be defined by a suitable qualified and/or experienced professional. An initialized machine-learning method is applied to each input data entry to generate predicted output data entries. An error between the predicted output data entries and corresponding training output data entries is used to modify the machine-learning method. This process can be repeated until the error converges, and the predicted output data entries are sufficiently similar (e.g. ±1%) to the training output data entries. This is commonly known as a supervised learning technique.

For example, where the machine-learning method is formed from a neural network, (weightings of) the mathematical operation of each neuron may be modified until the error converges. Known methods of modifying a neural network include gradient descent, backpropagation algorithms and so on.

The training input data entries correspond to example instances of medical imaging data and/or imaging system settings. The training output data entries correspond to changes to be made to the imaging system setting(s).

The machine-learning method may be specific for the anatomical feature(s) to be imaged in the medical imaging procedure and/or the purpose of the medical imaging procedure. This ensures that the medical imaging data output using the medical imaging system settings defined (using the machine-learning method) is suited for the particular medical imaging procedure.

**TABLE 1**

| Scenario | Randomly Initialized | Initialized with Previous Settings |
|---|---|---|
| No. of Iterations | 17 | 5 |
| Final Loss Value | 0.0089 | 0.0090 |

Table 1 illustrates the effect of the proposed approach when an iterative modification process is employed. In particular, Table 1 quantifies the number of iterations required for the image system settings to converge to a same/similar loss value for different initial value(s) of the image system settings. As shown in Table 1, the number of iterations is significantly reduced (e.g., a 70% improvement) if previous image system settings are identified and used.

Fig. 4 illustrates one approach for performing step 210 to obtain anatomical information.

In the illustrated example, step 210 comprises a sub-step 410 of obtaining, from the medical imaging system, initial imaging data of the subject. The initial imaging data may, for instance, have been produced using randomized or pseudo-randomized imaging system settings.

In the illustrated example, step 210 comprises a sub-step 420 of processing the initial imaging data to identify anatomical information that indicates one or more anatomical features of the subject represented in the initial imaging data. Sub-step 420 may, for instance, comprise processing the initial imaging data using a second machine-learning method (i.e., appropriately trained to produce anatomical information of a subject).

Approaches for processing imaging data to identify anatomical information are known in the art, and include one or more segmentation and/or classification techniques. For instance, an anatomical classification technique may be used to process the imaging data to predict the presence or absence of one or more anatomical elements represented in the imaging data and/or to identify an anatomical view of the imaging data.

A wide variety of approaches for processing imaging data to identify anatomical features or structures are known, and can be employed in some examples to produce anatomical information. Example approaches may make use of one or more machine-learning methods, e.g., neural networks.

Khan, Sameer, and Suet-Peng Yong. "A deep learning architecture for classifying medical images of anatomy object." 2017 Asia-Pacific Signal and Information Processing Association Annual Summit and Conference (APSIPA ASC). IEEE, 2017 discloses one approach for identifying the presence/absence of anatomical structures in imaging data. Roth, Holger R., et al. "Anatomy-specific classification of medical images using deep convolutional nets." 2015 IEEE 12th international symposium on biomedical imaging (ISBI). IEEE, 2015 discloses another technique. A wide variety of other approaches are known.

United States Patent No. US 11,468,567 discloses one approach for identifying an anatomical view provided by imaging data. European Patent Application having publication No. EP 4,136,617 A1 discloses another approach for identifying a view provided by imaging data.

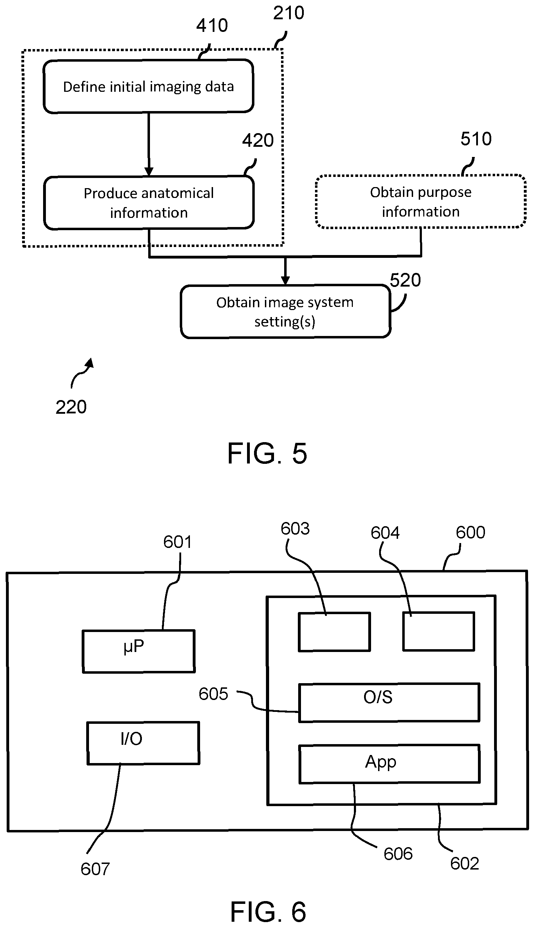

Fig. 5 illustrates an alternative approach for performing step 220.

In this approach, step 220 further comprises a sub-step 510 of obtaining purpose information that indicates a purpose for medical imaging data to be produced by the medical imaging system performing the medical imaging procedure. The purpose information may, for instance, contain a code or textual data.

The purpose information may be defined by a user input at a user interface. Accordingly, sub-step 510 may comprise obtaining, from a user interface, the purpose information.

Another approach for defining the purpose information is to process medical data and/or a medical record of the subject (to be imaged), e.g., to identify a purpose for a scheduled medical imaging procedure. For instance, there may be known or defined correlations or mappings between particular subject history information and a purpose for performing a medical imaging procedure.

By way of example, if a cancer is suspected (e.g., as indicated in notes of a medical record), then a purpose of a medical imaging procedure may be to identify any cancerous growths. As another example, if the subject has a high temperature and fast heart rate, then a purpose of the medical imaging procedure may be to identify the presence of an infection. As yet another example, if a stroke score (e.g., a score on the NIH stroke scale) was recently recorded and is below a predetermined score, then a purpose of the medical imaging procedure may be to identify the presence or absence of a stroke. A wide variety of other potential mappings between medical data and a purpose for a medical imaging procedure will be readily apparent to the skilled person.

The step 220 further comprises a sub-step 520 of using the anatomical information and the purpose information to obtain, from the database, one or more imaging system settings used by the medical imaging system to perform the previous medical imaging procedure, wherein the previous medical imaging procedure was performed on the same one or more anatomical features as represented in the initial imaging data and had a same purpose as the medical imaging procedure.

Thus, initial medical imaging system settings are based on those used for a previous medical imaging procedure that shared a same purpose as the upcoming medical imaging procedure. This significantly reduces a number of iterations required to converge the value(s) for the imaging system settings.

Of course, it is possible that no suitable imaging system setting(s) will be obtainable in step 210. This may occur, for instance, if this is the first time that the medical imaging procedure has been performed on the subject or no medical imaging procedure has been performed on the same anatomical region of the subject or no imaging system setting(s) were recorded for any such imaging procedures. As another example (if purpose information is used), no medical imaging procedure may have been performed on the subject for the same purpose.

In such circumstances, the imaging system settings may be simply selected randomly or pseudorandomly. In another example, the imaging system settings may be set based on default settings for the medical imaging procedure. In yet other examples, the imaging system settings may be set based on recommended settings for the subject based on demographic information of the subject. A wide variety of other procedures and processes will be readily apparent to the skilled person.

In some embodiments, if imaging system settings are available for the subject sharing the same anatomical information, but not sharing the same purpose information (where used), then the available system settings sharing the same anatomical information are instead obtained in step 220.

In some embodiments, if imaging system settings are available for the subject sharing the same purpose information (where used), but not sharing the same anatomical information, then the available system settings sharing the same purpose information are instead obtained in step 220.

In some embodiments, if imaging system settings are available for the subject but not sharing the same anatomical information and/or purpose information (where used), then the available system settings are instead obtained in step 220.

In some examples, if no imaging system settings are available for the subject, then the imaging system settings may be simply selected randomly or pseudorandomly, or based on default settings for the medical imaging procedure in step 220.

In previously described embodiments, only the step of obtaining image system settings makes use of the anatomical information and, if used, purpose information. However, in some further examples, the step of tuning the image system settings makes further use of the anatomical information and/or the purpose information.

In particular, the aim and/or of the tuning using the machine-learning method may differ responsive to information contained in the anatomical information and/or purpose information. In particular, the amount of tuning and/or selection of (available) settings that are tuned may change responsive to anatomical information and/or purpose information.

As an example, if the purpose information indicates that a purpose of the medical imaging procedure is to capture a disease progression, then image system settings that affect or change an amount of speckle in the medical imaging data should be unchanged from those of the previous medical imaging procedure. Changes in speckle represent an underlying change in the microstructure of target anatomy, which is caused by disease progression. The skilled person would be readily capable of identifying those settings that (if modified) would result in a change in the speckle of medical imaging data.

As another example, if the anatomy information and/or purpose information indicates that the medical imaging procedure is a fetal medical imaging procedure (e.g., the anatomy is a fetus or the purpose is obstetrics), then there may be no limitation on the number of imaging system settings or the amount of modification, as the underlying fetus is expected to change over different trimesters such that image quality is a higher priority.

In the context of the present invention, an amount of modification is a quantification of the change to each image system setting performed by step 230, e.g., expressible as a percentage.

Other examples will be apparent to the skilled person depending upon the particular use-case scenario for the medical imaging procedure.

Thus, referring back to Fig. 2, the method 200 may be adapted wherein the step 230 of tuning the image system setting(s) comprises further using the anatomical information and/or purpose information to perform the tuning. In particular, the anatomical information and/or purpose information may define the image system settings that can be tuned or modified in step 230 and/or be provided as input to the machine-learning method used to perform the tuning and/or modification to the image system settings (e.g., to control the extent to which tuning is performed).

Put another way, the step of tuning the obtained one or more image system settings may further comprise using the anatomical information and/or the purpose information to define the number of image system settings that are modified and/or the amount to which the image system settings are modified using the machine-learning method.

In particular, the tuning performed in step 230 may aim to balance between consistency across the previous and the present medical imaging procedure and image quality of the present medical imaging procedure (depending upon the precise use-case scenario defined by the anatomical information and/or purpose information).

Put another way, step 230 may comprise weighting the tuning of the image system settings responsive to the anatomical information and/or purpose information. The weighting may represent a desired outcome for a particular use-case scenario defined by the anatomical and/or purpose information. The exact weighting will, of course, vary depending upon the exact embodiment as would be readily apparent to the skilled person.

More specifically, if the purpose of the medical imaging procedure is to perform a follow-up to an earlier medical imaging procedure (i.e., to determine a progression of a particular etiology or undesirable anatomical feature (e.g., tumor or growth) and/or disease), then the number of image system settings modified and/or the amount to which the image system settings are modified may be lower than if the purpose of the medical imaging procedure is not to perform a follow-up to an earlier medical imaging procedure (e.g., for obstetrics or any other procedure that does not investigate the progression of an etiology or undesirable anatomical feature and/or disease).

The skilled person would be readily capable of developing a processing system for carrying out any herein described method. Thus, each step of the flow chart may represent a different action performed by a processing system, and may be performed by a respective module of the processing system.

Fig. 6 illustrates one example of a suitable processing system 600.

Various operations discussed above may utilize the capabilities of the computer 600. For example, one or more parts of a processing system for modifying imaging system settings (of a medical imaging system) may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 601, memory 602, and one or more I/O devices 607 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 601 is a hardware device for executing software that can be stored in the memory 602. The processor 601 can be virtually any custom made or commercially available processor, a central processing unit (CPU), a digital signal processor (DSP), or an auxiliary processor among several processors associated with the computer 600, and the processor 601 may be a semiconductor based microprocessor (in the form of a microchip) or a microprocessor.

The memory 602 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 602 may incorporate electronic, magnetic, optical, and/or other types of storage media. Note that the memory 602 can have a distributed architecture, where various components are situated remote from one another, but can be accessed by the processor 601.

The software in the memory 602 may include one or more separate programs, each of which comprises an ordered listing of executable instructions for implementing logical functions. The software in the memory 602 includes a suitable operating system (O/S) 605, compiler 604, source code 603, and one or more applications 606 in accordance with exemplary embodiments. As illustrated, the application 606 comprises numerous functional components for implementing the features and operations of the exemplary embodiments. The application 606 of the computer 600 may represent various applications, computational units, logic, functional units, processes, operations, virtual entities, and/or modules in accordance with exemplary embodiments, but the application 606 is not meant to be a limitation.

The operating system 605 controls the execution of other computer programs, and provides scheduling, input-output control, file and data management, memory management, and communication control and related services. It is contemplated by the inventors that the application 606 for implementing exemplary embodiments may be applicable on all commercially available operating systems.

Application 606 may be a source program, executable program (object code), script, or any other entity comprising a set of instructions to be performed. When a source program, then the program is usually translated via a compiler (such as the compiler 604), assembler, interpreter, or the like, which may or may not be included within the memory 602, so as to operate properly in connection with the O/S 605. Furthermore, the application 606 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 607 may include input devices such as, for example but not limited to, a mouse, keyboard, scanner, microphone, camera, etc. Furthermore, the I/O devices 607 may also include output devices, for example but not limited to a printer, display, etc. Finally, the I/O devices 607 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 607 also include components for communicating over various networks, such as the Internet or intranet.

If the computer 600 is a PC, workstation, intelligent device or the like, the software in the memory 602 may further include a basic input output system (BIOS) (omitted for simplicity). The BIOS is a set of essential software routines that initialize and test hardware at startup, start the O/S 605, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 601 is configured to execute software stored within the memory 602, to communicate data to and from the memory 602, and to generally control operations of the computer 600 pursuant to the software. The application 606 and the O/S 605 are read, in whole or in part, by the processor 601, perhaps buffered within the processor 601, and then executed.

When the application 606 is implemented in software it should be noted that the application 606 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 606 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

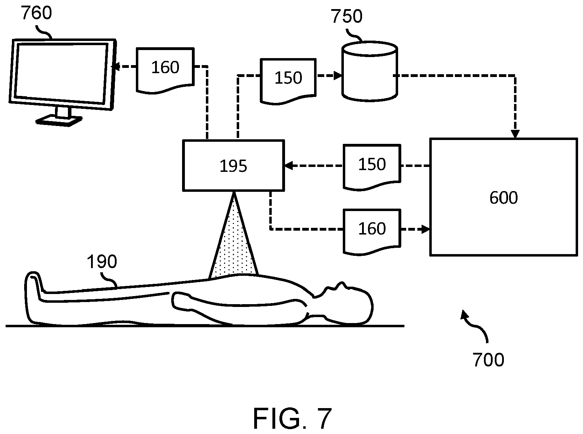

Fig. 7 illustrates an imaging system 700 according to an embodiment.

The imaging system 700 comprises a processing system 600, which may be embodied as previously described. The imaging system 700 also comprises the medical imaging system 195 configured to use the one or more imaging system settings 150 defined by the processing system to perform the medical imaging procedure and generate medical imaging data 160.

The medical imaging system 195 may, for instance, be an ultrasound imaging system comprising an ultrasound transducer for capturing (as the medical imaging data) ultrasound imaging data or medical ultrasound data.

In some examples, the imaging system is further configured to store, in the database, the one or more imaging system settings used by the medical imaging system to perform the medical imaging procedure and generate the medical imaging data.

Accordingly, the imaging system 700 may further comprise the database 750.

The imaging system 700 may further comprise a user interface 760 configured to display a visual representation of any generated medical imaging data 160. Approaches for controlling a user interface 760 to provide a display of the visual representation of the generated medical imaging data 160 are known.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. A computer-implemented method (200) for defining one or more imaging system settings (150) for a medical imaging system (195) to use in performing a medical imaging procedure on a subject (190), the computer-implemented method comprising:
obtaining (210) anatomical information that indicates one or more anatomical features of the subject to undergo the medical imaging procedure;
obtaining (220), from a database and using the anatomical information, one or more imaging system settings used to perform a previous medical imaging procedure on the indicated one or more anatomical features of the subject; and
tuning (230) the obtained one or more image system settings using a machine-learning method.

2. The computer-implemented method (200) of claim 1, wherein the step of tuning the obtained one or more image system settings comprises performing an imaging system setting modification process comprising:
obtaining (321), from the medical imaging system, medical imaging data obtained using the one or more imaging system settings;
processing (322) the medical imaging data, using the machine-learning method, to identify one or more changes to be made to the one or more imaging system settings; and
modifying (323) the one or more imaging system settings using the identified one or more changes.

3. The computer-implemented method of claim 2, wherein the step of tuning the obtained one or more image system settings comprises iteratively performing the imaging system setting modification process until one or more termination conditions are met.

4. The computer-implemented method of claim 3, wherein the one or more termination conditions include:
a quality measure of the medical imaging data meeting one or more predetermined conditions;
the imaging setting modification process being performed no less than a predetermined number of times;
a measure of a difference between the imaging system settings for a current iteration of the imaging setting modification process and the imaging system settings for an immediately previous iteration of the imaging setting modification process being less than a predetermined value; and/or
an override indicator being provided.

5. The computer-implemented method of any of claims 1 to 4, wherein the step of obtaining anatomical information comprises:
obtaining, from the medical imaging system, initial imaging data of the subject; and
processing the initial imaging data to identify the anatomical information.

6. The computer-implemented method of claim 5, wherein the initial imaging data is obtained by the medical imaging system using randomized or pseudo-randomized imaging system settings.

7. The computer-implemented method of any of claims 5 or 6, wherein the step of processing the initial imaging data comprises processing the initial imaging data using a second machine-learning method to generate the anatomical information.

8. The computer-implemented method of any of claims 1 to 7, wherein the step of obtaining, from a database, one or more imaging system settings comprises:
obtaining purpose information that indicates a purpose for medical imaging data to be produced by the medical imaging system performing the medical imaging procedure; and
using the purpose information and the anatomical information to obtain, from the database, one or more imaging system settings used by the medical imaging system to perform the previous medical imaging procedure,
wherein the previous medical imaging procedure had a same purpose as the medical imaging procedure and was performed on the same one or more anatomical features as represented in the anatomical information.

9. The computer-implemented method of claim 8, wherein the step of tuning the obtained one or more image system settings further comprises using the purpose information to define the number of image system settings that are modified and/or the amount to which the image system settings are modified using the machine-learning method.

10. The computer-implemented method of any of claims 1 to 9, wherein the medical imaging system is an ultrasound imaging system and the medical imaging data is medical ultrasound data.

11. A computer program product comprising computer program code means which, when executed on a computing device having a processing system, cause the processing system to perform all of the steps of the method according to any of claims 1 to 10.

12. A processing system for defining one or more imaging system settings for a medical imaging system to use in performing a medical imaging procedure on a subject, the processing system being configured to:
obtain anatomical information that indicates one or more anatomical features of the subject to undergo the medical imaging procedure;
obtain, from a database and using the anatomical information, one or more imaging system settings used to perform a previous medical imaging procedure on the indicated one or more anatomical features of the subject; and
tune the obtained one or more image system settings using a machine-learning method.

13. An imaging system comprising:
the processing system of claim 12; and
the medical imaging system configured to use the one or more imaging system settings defined by the processing system to perform the medical imaging procedure and generate medical imaging data.

14. The imaging system of claim 13, wherein the medical imaging system is an ultrasound imaging system comprising an ultrasound probe configured to capture, as the medical imaging data, medical ultrasound data.

15. The imaging system of claim 13 or 14, further configured to store, in the database, the one or more imaging settings used by the medical imaging system to perform the medical imaging procedure and generate the medical imaging data.
